# EUROPEAN PATENT SPECIFICATION

(11) **EP 0 400 035 B1**
(45) Date of publication and mention of the grant of the patent: **10.08.1994**
(21) Application number: 89902092.9
(22) Date of filing: 27.01.1989
(51) Int. Cl.: A61F 2/30, A61B 17/58

(54) **DEVICE AT ARTIFICIAL JOINTS**
ANORDNUNG AN KUNSTGLIEDERN
DISPOSITIF POUR ARTICULATIONS ARTIFICIELLES

(30) Priority: 28.01.1988 SE 8800274
(43) Date of publication of application: 05.12.1990
(73) Proprietor: Unilink Inc., Rockville, Maryland 20850 (US)
(72) Inventor: BERGGREN, Anders, S-582 45 Linköping (SE); ROHMAN Hakan, S-590 20 Mantorp (SE)
(74) Representative: Berglund, Erik Wilhelm
(86) International application number: PCT/SE89/00025
(87) International publication number: WO 89/06946

(56) References cited:
- EP-A- 0 024 008
- DE-A- 3 536 894
- DE-A- 3 613 657
- US-A- 4 064 567

## Description

This invention concerns a fastening means for fastening a bone part to an artificial part or to an artificial joint part comprising an elongated supporting structure which is insertable into a bone and comprising bristles, fibres, loops or the like radially extending from the supporting structure with such a length that the outer diameter is greater than that of the bone cavity into which the supporting structure is intended to be inserted so that the bristles, fibres or loops receive a certain pretensioning at the mounting angling these away from the direction of insertion.

To replace worn or in other way for instance as a result of rheumatism damaged joints it is since considerably long time known to remove a joint end from a bone and replace this end with an artificial part. These artificial parts include a joint part and a connection part that is inserted into the cavity of the remaining bone. The fastening in the bone has been carried out in different ways for instance the connection part has been threaded and/or cemented into the bone.

To start with this is comparatively complicated and time consuming and secondly great loads as for instance in the vicinity of knee joints and tigh bones with time give an increasing play between artificial part andbone, which in turn result in a necessary change of the artificial part, which also normally includes the shortening of the bone to obtain sufficiently good material to fasten the artificial part. After a few changes of the artificial part so little of the bone will remain that the joint has to be made rigid due to the lack of bone material.

In the European patent application EP-A-0024008 is described an artificial tooth that tries to resemble a real tooth as much as possible by being provided with fibres extending upwards from a central part so that they can function in the same way as the so called Sharpey's fibre that exists around the natural tooth. In this way no pressure forces are excerted but only pulling forces and it is thus possible for the tooth to be elastically mounted. The technique of this citation does however not indicate any practical way to obtain an artificial part that can be mounted into a bone and that immediately after mounting is unyieldingly mounted in the bone.

A further drawback of the known artificial parts this far is that the inner cavity of the bone is not available for the formation of vessels in a natural way, which presumably further reduces the life spann of the mounting of an artificial part and thus of the artificial part itself.

The invention has as its object to solve the above mentioned problem and provide a simple useful artificial part fastening or mounting that is easy to apply, that does not need to be exchanged and that allows an improved bleeding. This is in accordance with the invention achieved by the supporting structure being constituted by two wires wound around each other and that the bristles are clamped between the wound wires.

When the fastening element is inserted into the hollow of the bone the bristles are bent somewhat which will give a greater resistance against withdrawal than against the insertion. It will in fact be practically impossible to withdraw the fastening element. Preferably the number of bristles, fibres or the corresponding is very great. In this way a very great contact surface between bone and artificial part fastening element is obtained simultaneously as an adaption is automatically made to possible irregularities on the inside of the bone.

By furthermore fabricating the bristles, fibres or loops in titanium the bone and the artificial part fastening element will grow together and will so to say become integrated with time. Bleeding and the development of vessels can take place between the bristles or loops and the supporting structure is more or less porous or open. Since the tensions are less further into the bone the fastening element can there be more open or be provided with less bristles. The length of the bristles and the density thereof are above all ruled by the forces that has to be transfered and the density of the bristles need not necessarily be the same over the entire length of the fastening element.

If bristles of titanium is used, thin but many bristles can be used in view of the hard and stiff character of titanium. Instead of titanium however titanium containing alloys coated by titanium can be used and it is also possible to consider plastic materials coated with titanium.

The bristles can from the start be directed radially or possibly somewhat rearwards or it is of course possible that only some of them are.

The supporting structure need not necessary be rigid but may allow a certain elasticity that may preferably be calculated to coincide with that of the bone so that no tension peaks are developed in the force transfer forth and back.

By means of the invention an immediate sturdy grip is obtained in the bone preventing the mounted artificial part from loosening. With the use of titanium the device is prevented from working loose with time since the contact surface is great and thus the specific load small.

It should be observed that the fastening means described above can also be used for the jointing of broken bones.

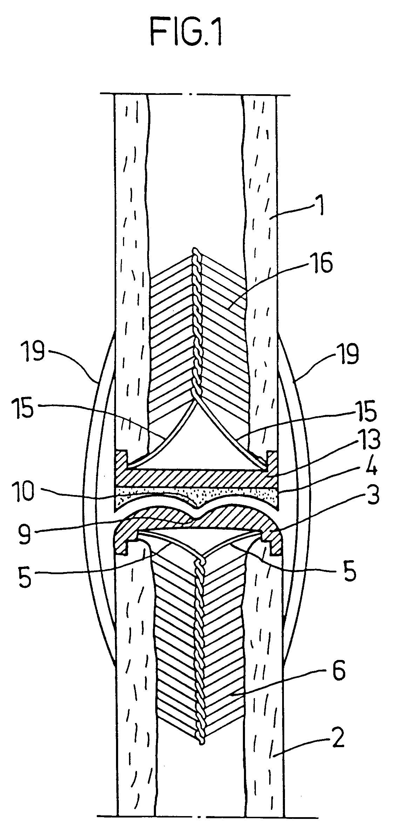

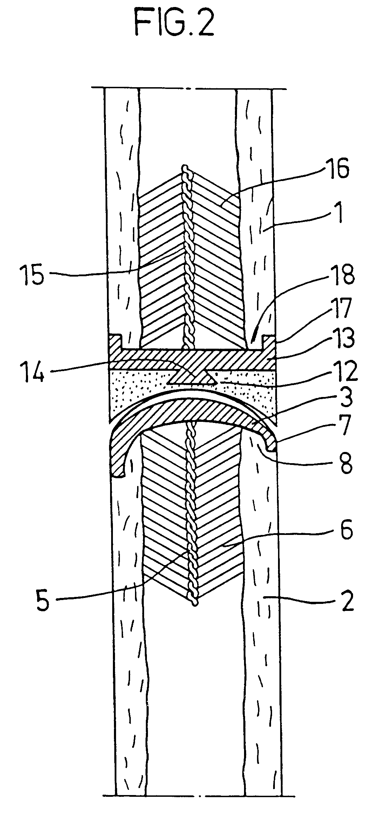

Further details and advantages of the invention are apparent from the subclaims and the following description of a prefered embodiment and the claims. Fig 1 shows a joint seen from the front and fig 2 the same joint seen from the side, both in a longitudinal section.

The artificial joint shown in the drawings includes a bowl or concave joint part 4 and a ball concave joint part 3 each fastened in a bone 1 and 2 respectively. The lower part, the ball part 3 is welded to two wires 5 of titanium which from diametrical peripherial fastening points extend towards each other, then to continue in a winding around each other. Between the windings of these two wires 5 thin threadlike bristles 6, also of titanium are clamped.

The bristles 6 have such a length that they on insertion into the bone 2 will be bent somewhat upwards. This means that they will not excert any particular resistance against the introducing into the bone. However the resistance against possible attempts to withdraw the artificial joint part from the bone will be very great. By using a great number of bristles a good fastening against a turning of the artificial joint part relative the bone is achieved.

The ball joint part 3 has a circumferencal downwards protruding flange 7 gripping around the bone 2 on it's correspondingly shaped end 8. In this way the artificial joint part will be locked against possible tilting with the bristles and the titanium wires pressing the ball joint part against the bone 2.

Possibly the inner of the ball joint part 3 can be filled with silicon at the mounting alternatively can the inner be entirely empty. By useing pure titanium or titanium alloys or other materials coated with titanium or perhaps even other materials which can grow together with the bone an integration is achieved with time. In this way not only the immediate sturdy fixing is obtained but also a long time fixing.

The upper joint bowl part or concave part 4 has a shape corresponding to the shape of the ball joint part 3 so that an angular movement is possible. The surfaces turned against each other has principally the shape of cylinder or sphere surfaces where the lower ball joint part 3 has a waist or groove 9 into which a protrusion or ridge 10 on the upper bowl joint part can grip to provide an increased resistance against turning and dislocation sidewise. By dimensioning the interrelated length of groove and ridge angle stops for the joint can be achieved. The upper bowl joint part 4 is not made of titanium but is made of a synthetic material that against titanium gives a low friction. An example of this is HDP (high density polyethylen) that has shown itself to be a material that work well with human tissues. In the bowl part 4 a dove-tail groove 12 extends sidewise and into this grips a correspondingly shaped dove-tail 14 that in turn is a part of a titanium plate 13. Preferably the titanium plate and the joint bowl part 3 of HDP are provided with snap means so that the lateral location when well achieved is secured. To the plate 13 is then welded wires 15 which are then wound to a spiral that holds bristles 16 in the same way as has been described above for the lower ball shaped joint part. Also the titanium disk or plate 13 is provided with a flange 17 gripping around a corresponding shaping 18 of the bone 1.

By use of a dove-tail joint to join the joint ball part with it's fastening means the advantage is obtained that with comparatively simple means the joint bowl part can easily be exchanged if it for instance after many years of use becomes too worn and this with an operation that is far easier and less complicated and time consuming than would be the case if one would have to exchange the entire artificial joint or artificial joint part.

Of course also the lower ball joint part can be made in a corresponding way to allow a change of the part subjected to wear. This can for instance take place by means of a dove-tail joint with the interlocation of a plastic part. By using plastic for one half of the joint and possibly as a joining element in the lower part a certain elasticity against shock is achieved which reduce tensions in other joints as well as in the fastening of the artificial joint.

It should be observed that the flanges 7, 17 and the corresponding shaping of the bone ends respectively need not necessarily be exactly after the outer shape of the bone, but to fasciliate working it is of course also possible to use flanges that are circular for instance concentric with the axis of the wound titanium wires.

The joint has on each side a ligament 19.

By means of the invention an artificial joint or joint parts is achieved for big as well as small joints that are very similar to the desired relationships of the original joint.

Since the fastening according to the invention in principle is or can be made elastic over its entire length there will be no tension peaks where the artificial part ends which is a regretfully case with many known artificial joint parts.

Due to the improved fastening characteristics and the soft transfering of forces from the artificial part to the bone it is actually possible to make replacements of another character than has previously been possible. Due to the previously poor fastening characteristics the artificial part has always had to be comparatively short in order not to have too great torques excerted on the remaining bone part. With the invention the remaining bone will not be subjected to forces greater than the bone originally could take. Therefore an artificial joint part according to the invention can be longer which in turn means that the previous shortening of bones will become unnecessary.

In the drawings has been shown the use of two wires for fastening the bristles. More wires than two can of course be used and it is also possible to use a supporting structure carrying the bristles that are shaped in another way. For instance the bristles could be fastened in a cast plastic structure.

If the supporting structure has a stiffness that reduces towards the inner of the cavity the transfering of forces from and to the joint will take place without significant tension peaks.

In the above example two wires have been used to clamp or fasten the bristles, of course a greater number of bristles can also be used. Furthermore if an-other type of supporting structure of for instance plastic is used a porous or open structure can also be employed.

## Claims

1. Fastening means for fastening a bone part to an artificial part or to an artificial joint part comprising an elongated supporting structure (5, 15) which is insertable into a bone (1, 2) and comprising bristles (6, 16), fibres, loops or the like radially extending from the supporting structure with such a length that the outer diameter is greater than that of the bone cavity into which the supporting structure (5, 15) is intended to be inserted so that the bristles (6, 16), fibres or loops receive a certain pretensioning at the mounting angling these away from the direction of insertion, **characterized in** that the supporting structure is constituted by two wires (5, 15) wound around each other and that the bristles (6, 16) are clamped between the wound wires.

2. Means according to claim 1, **characterized in** that the bristles (6, 16), or fibres or loops are made of titanium or alloys thereof or other materials coated with titanium.

3. Means according to claim 1 or 2, **characterized in** that the means comprises an artificial joint part (3, 4), the artificial joint part including flanges (7, 17) or protrusions that are to abut against the end of the bone (1, 2).

4. Means according to any of the previous claims, **characterized in** that the means comprises an artificial joint part (3, 4), the artificial joint part consisting of a ball joint part (3) or a concave part (4).

5. Means according to claim 4, **characterized in** that the bearing surfaces of the joint is securable to the fastening means of a dovetail joint (12, 14).

6. Means according to claim 5, **characterized in** that the dovetail joint (12, 14) extends perpendicular to the bending plane of the joint.

7. Means according to claim 6, **characterized in** that the number of bristles (6, 16), fibres or loops are great.

## Patentansprüche

1. Befestigungsmittel zur Befestigung eines Knochenteils an einem künstlichen Teil oder an einem künstlichen Gelenkteil, mit einem länglichen Halter (5, 15), der in den Knochen (1, 2) einschiebbar ist und Borsten (6, 16), Fasern, Maschen oder dergleichen aufweist, die vom Halter radial nach aussen weisen und so lang sind, dass der Aussendurchmesser grösser ist als derjenige des Knochenhohlraums, in welchen der Halter (5, 15) eingeschoben werden soll, so dass die Borsten (6, 16), Fasern oder Maschen bei der Befestigung eine gewisse Vorspannung erhalten, durch welche sie entgegen der Einschubrichtung umgebogen werden, dadurch gekennzeichnet, dass der Halter aus zwei Drähten (5, 15) besteht, welche miteinander verdreht sind, und dass die Borsten (6, 16) zwischen den verdrehten Drähten festgeklemmt sind.

2. Mittel nach Anspruch 1, dadurch gekennzeichnet, dass die Borsten (6, 16), Fasern oder Maschen aus Titan oder Titanlegierungen oder aus anderen titanbeschichteten Materialien bestehen.

3. Mittel nach Anspruch 1 oder 2, dadurch gekennzeichnet, dass es einen künstlichen Gelenkteil (3, 4) besitzt, welcher Flansche (7, 17) oder Fortsätze aufweist, die dazu vorgesehen sind, am Knochenende (1, 2) anzuliegen.

4. Mittel nach einem der vorstehenden Ansprüche, dadurch gekennzeichnet, dass es einen künstlichen Gelenkteil (3, 4) aufweist, der aus einem Gelenkkugelteil (3) oder einem konkaven Teil (4) besteht.

5. Mittel nach Anspruch 4, dadurch gekennzeichnet, dass die Auflagefläche des Gelenks mittels einer Schwalbenschwanzverbindung (12, 14) am Befestigungsmittel befestigbar ist.

6. Mittel nach Anspruch 5, dadurch gekennzeichnet, dass die Schwalbenschwanzverbindung (12, 14) senkrecht zur Beugungsebene des Gelenks verläuft.

7. Mittel nach Anspruch 6, dadurch gekennzeichnet, dass die Anzahl von Borsten (6, 16), Fasern oder Maschen gross ist.

## Revendications

1. Moyen de fixation pour la fixation d'une partie osseuse à une partie artificielle ou une partie d'articulation artificielle, comprenant une structure de support (5, 15) allongée qui s'insère dans un os (1, 2) et qui présente des crins (6, 16), fibres, mailles ou autres s'étendant radialement de la structure de support et ayant une telle longueur que le diamètre extérieur est plus grand que celui de la cavité de l'os dans laquelle la structure de support (5, 15) doit être insérée, de sorte que les crins (6, 16), fibres ou mailles reçoivent lors du montage une certaine précontrainte qui les plie dans le sens opposé à la direction d'insertion, caractérisé en ce que la structure de support est constituée de deux fils (5, 15) tordus l'un autour de l'autre, et que les crins (6, 16) sont serrés entre les fils tordus.

2. Moyen selon la revendication 1, caractérisé en ce que les crins (6, 16), fibres ou mailles sont faits de titane ou d'alliages de titane, ou d'autres matériaux revêtus de titane.

3. Moyen selon la revendication 1 ou 2, caractérisé en ce qu'il comporte une partie d'articulation artificielle (3, 4) qui présente des collets (7, 17) ou projections venant s'appuyer sur l'extrémité de l'os (1, 2).

4. Moyen selon l'une quelconque des revendications précédentes, caractérisé en ce qu'il comporte un partie d'articulation artificielle (3, 4) qui consiste d'une partie en tête d'articulation (3) ou d'une partie concave (4).

5. Moyen selon la revendication 4, caractérisé en ce que la surface d'appui de l'articulation est attachable au moyen de fixation par un joint en queue d'aronde (12, 14).

6. Moyen selon la revendication 5, caractérisé en ce que le joint en queue d'aronde (12, 14) s'étend perpendiculairement au plan de flexion de l'articulation.

7. Moyen selon la revendication 6, caractérisé en ce que le nombre de crins (6, 16), fibres ou mailles est elevé.
